**Europäisches Patentamt**

**European Patent Office**

**Office européen des brevets**

(19)

(11) Veröffentlichungsnummer: **0 220 479**
**B1**

(12) ## EUROPÄISCHE PATENTSCHRIFT

(45) Veröffentlichungstag der Patentschrift:
**07.02.90**

(21) Anmeldenummer: **86112927.8**

(22) Anmeldetag: **18.09.86**

(51) Int. Cl.⁴: **A61F 7/00**, F25B 19/00,
F25D 3/10

(54) **Kabine zur Durchführung der Kryotherapie.**

(30) Priorität: **28.09.85 DE 3534630**

(43) Veröffentlichungstag der Anmeldung:
**06.05.87 Patentblatt 87/19**

(45) Bekanntmachung des Hinweises auf die Patenterteilung:
**07.02.90 Patentblatt 90/6**

(84) Benannte Vertragsstaaten:
**AT BE CH FR GB IT LI NL SE**

(56) Entgegenhaltungen:
**AT-B- 376 032**
**DE-A- 3 242 881**
**US-A- 3 885 571**

(73) Patentinhaber: **MESSER GRIESHEIM GMBH, Hanauer Landstrasse 330, D-6000 Frankfurt/Main 1(DE)**

(72) Erfinder: **Donnerhack, Andreas, Dr., Bismarckstrasse 4, D-4150 Krefeld(DE)**
Erfinder: **Thoma, Klemens, Am Kleckers 22, D-4150 Krefeld-Hüls 29(DE)**
Erfinder: **Volker, Wolfgang, Pastorsbusch 35, D-4154 Tönisvorst 1(DE)**
Erfinder: **Gallmeister, Rolf-Dieter, Uhlandstrasse 14, D-7880 Bad Säckingen(DE)**
Erfinder: **Stratz, Thomas, Dr., Purkersdorferstrasse 49, D-7880 Bad Säckingen(DE)**

## Beschreibung

Die Erfindung betrifft eine Kabine zur Durchführung der Kryotherapie am ganzen Körper mit einem kalten Behandlungsgas, nach dem Oberbegriff des Anspruches 1.

Neben der seit einigen Jahren durchgeführten lokalen Kryotherapie mit einem kalten Behandlungsgas, beispielsweise zur Behandlung rheumatischer Erkrankungen, wird bei bestimmten Krankheitsformen auch eine Kryotherapie am ganzen Körper durchgeführt. Hierbei wird mit Hilfe von flüssigem Stickstoff Luft in Wärmetauschern abgekühlt und in eine geschlossene Kammer eingedüst. Diese Kammer oder Kabine besitzt Wände aus isolierendem Material und Anschlüsse zur Zufuhr und Ableitung des Behandlungsgases. Eine solche Kammer zeigt beispielsweise das japanische Gebrauchsmuster Nr. 168 125/81. Dieses Konzept findet jedoch sowohl bei Ärzten als auch bei den Patienten wenig Anklang. Die Gründe dafür sind vielfältig. Die Patienten beanstanden den fehlenden direkten Kontakt zum Arzt, da während der Behandlung nur ein indirekter Kontakt durch Fenster und über Sprecheinrichtungen möglich ist. Die starke Nebelbildung in der Kammer verstärkt noch diesen Eindruck des mangelnden direkten Kontaktes. Nachteilig ist auch die unerwünschte Abkühlung im Kopfbereich des Patienten. Außerdem muß durch besondere Maßnahmen das Einatmen kalter Luft verhindert werden. Hiervon abgesehen, erfordern solche Kammern auch hohe Investitionskosten. Wegen der langen Anlaufzeiten besteht eine Notwendigkeit zum Dauerbetrieb, wodurch verhältnismäßig hohe Betriebskosten entstehen. Die Patientenüberwachung während der Behandlung ist aufwendig.

Der Erfindung liegt daher die Aufgabe zugrunde, eine Kabine zur Durchführung der Kryotherapie am ganzen Körper mit einem kalten Behandlungsgas zu schaffen, welche einen direkten Kontakt zwischen Arzt und Patienten während der Behandlung ermöglicht, den Kopf des Patienten freiläßt und wegen kurzer Anlaufzeiten keinen Dauerbetrieb erforderlich macht.

Ausgehend von dem im Oberbegriff des Anspruches berücksichtigten Stand der Technik ist diese Aufgabe erfindungsgemäß gelöst mit den im kennzeichnenden Teil des Anspruches 1 angegebenen Merkmalen.

Vorteilhafte Weiterbildungen der Erfindung sind in den Unteransprüchen angegeben.

Zwei Ausführungsbeispiele der Erfindung sollen anhand der beigefügten Zeichnungen erläutert werden.

Es zeigen:

Fig. 1 eine teilweise aufgebrochene perspektivische Ansicht einer Kabine mit verstellbaren Wänden,

Fig. 2 einen Schnitt durch eine Wand, der Kabine nach Fig. 1,

Fig. 3 einen Schnitt durch die Wand einer Kabine mit höhenverstellbarem Boden,

Fig. 4 eine Einrichtung zur Erzeugung des kalten Behandlungsgases, welche einen kostengünstigen Leerlaufbetrieb der Kabinen ermöglicht.

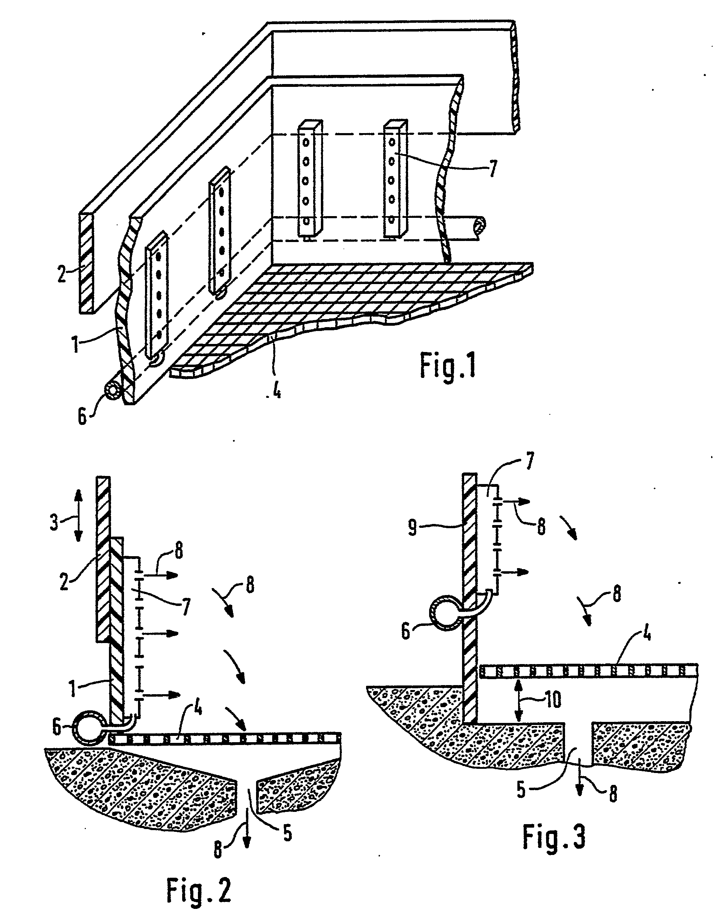

Die in den Figuren 1 und 2 dargestellte Kabine ist gemäß der Erfindung oben offen und besitzt höhenverstellbare Wände. Die Wand 1 steht fest und besitzt eine nicht dargestellte Tür zum Betreten der Kabine. Die Wand 1 besteht aus isolierendem Material, beispielsweise aus einer doppelwandigen Isolierverglasung aus Kunststoff. Die Wand 2 bildet den oberen Teil der Kabine und kann in vertikaler Richtung gleitend auf der Wand 1 verschoben werden. Die Richtung dieser Verschiebung ist durch einen Pfeil 3 angegeben. Auch die Wand 2 besitzt eine nicht dargestellte Tür zum Betreten der Kabine und ist ebenfalls aus isolierendem Material dargestellt. Die Wand 2 kann auf jede beliebige Art verschoben werden, im einfachsten Fall mechanisch von Hand, wobei sie in verschiedenen Höhen arretiert werden kann. Die Höhenverstellung kann jedoch auch aufwendiger, hydraulisch oder elektrisch, erfolgen.

Für die Behandlung wird die Wand 2 so verschoben, daß ihre Oberkante mit dem Hals des Patienten abschließt. Den Boden der Kabine bildet ein Gitterrost 4. Unter dem Gitterrost befindet sich eine Absaugöffnung 5. Die Zufuhr des kalten Behandlungsgases erfolgt durch eine Ringleitung 6 in mehrere Düsenleisten 7, die gleichmäßig über den Umfang der Kabine senkrecht angeordnet sind. Das Behandlungsgas wird durch die Absaugöffnung 5 abgesaugt. Die Strömungsrichtung ist durch Pfeile 8 angegeben.

Durch die Düsenleisten 7 wird der Kaltwind auf den Patienten gerichtet in die Kabine eingeleitet. Die Absaugung am Boden der Kabine sorgt dafür, daß der Kaltwindstrom nach unten gerichtet ist. Er strömt also nicht nach oben in den Raum und verursacht dort keine Nebelbildung. Von großem Vorteil ist dabei, daß der Kopf des Patienten nicht mit kaltem Behandlungsgas angeströmt wird. Während der Behandlung ist jederzeit ein direkter Sprechkontakt- und Sichtkontakt zwischen Arzt und Patienten möglich.

Die in Fig. 3 dargestellte Ausführungsform unterscheidet sich von der in den Figuren 1 und 2 dargestellten lediglich dadurch, daß hier der Gitterrost 4 höhenverstellbar ist und dafür nur eine feststehende Wand 9 vorgesehen ist. Die Möglichkeit der Höhenverstellung des Gitterrostes 4 ist durch einen Pfeil 10 angedeutet. Auch hier kann im einfachsten Fall die Höhenverstellung mechanisch von Hand erfolgen, natürlich können auch aufwendigere Mechanismen verwendet werden.

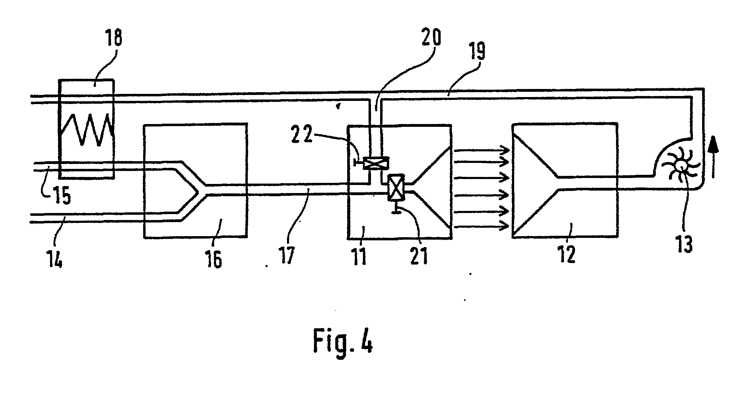

Für die Bildung des kalten Behandlungsgases können alle hierfür gebräuchlichen Einrichtungen und Gaszusammensetzungen verwendet werden. Vorteilhaft ist die Bildung des Behandlungsgases durch Vermischen von trockener Luft mit einem kalten verflüssigten Gas, vorzugsweise Stickstoff. Diese ermöglicht in besonders einfacher Weise einen kostengünstigen Leerlaufbetrieb der Kabine, wie in Fig. 4 dargestellt ist. Darin ist die Kabine schematisch dargestellt, bestehend aus einem Eintrittsteil 11 und einem Austrittsteil 12. Der Eintrittsteil 11 entspricht dabei den Wänden 1, 2 mit den Düsenleisten 7, der Austrittsteil 12 entspricht dem Gitterrost 4 mit der Absaugöffnung 5. Zur Zurückführung des Behandlungsgases dient das Absauggebläse 13, die Richtung der Gasströmung ist durch nicht gekennzeichnete Pfeile dargestellt. Das kalte Behandlungsgas wird gebildet aus flüssigem Stickstoff, der durch die Leitung 14 zugeführt wird, und trockener Luft, welche durch die Leitung 15 zugeführt wird. Die Bildung des Behandlungsgases aus diesen Komponenten erfolgt im Mischgerät 16, von wo aus es durch die Leitung 17 dem Eintrittsteil 11 zugeführt wird. Die Leitung 17 entspricht hierbei der Ringleitung 6 in den Figuren 1 bis 3. Vor dem Eintritt in das Mischgerät 16 durchströmt die Luft einen Wärmetauscher 18. Der Wärmetauscher 18 wird auch von dem durch Leitung 19 zurückströmenden abgesaugten Behandlungsgas durchströmt, so daß die eintretende Luft vorgekühlt wird.

Im Leerlaufbetrieb, wenn also gerade kein Patient behandelt wird, die Anlage aber betriebsbereit gehalten wird, wird das erzeugte kalte Behandlungsgas über Leitung 20 unmittelbar zurück in den Wärmetauscher 18 geleitet, ohne daß es in das Innere der Kabine eintritt. Die Umschaltung des Gasstromes wird mit den Ventilen 21 und 22 vorgenommen. Durch den kostengünstigen Leerlaufbetrieb wird bei kurzen Behandlungsunterbrechungen Stickstoff eingespart. Andererseits ist die Kabine nach derartigen Betriebspausen rasch wieder einsatzbereit. Es ist auch möglich, bei Leerlauf nur eine reduzierte Menge an Behandlungsgas zu erzeugen, um die Kaltwindanlage und die Zuleitungen kalt zu halten.

Außer der Energieeinsparung hat eine solche Betriebsweise auch den Vorteil, daß der Patient nicht unter Kühlbedingungen positioniert werden muß. Bei Behandlungsbeginn werden die Ventile 21, 22 umgeschaltet, so daß das Behandlungsgas aus den Düsenleisten 7 in das Innere der Kabine eintritt.

Die Kabine ist mit den üblichen Sicherheitseinrichtungen ausgerüstet, die jedoch nicht dargestellt sind. Es handelt sich hierbei im wesentlichen um einen Sauerstoff-Sensor im Kopfbereich des Patienten, der bei Sauerstoffmangel in der Atemluft automatisch eine Abschaltung des Kühlbetriebes auslöst. Des weiteren können Infrarot-Sonden vorgesehen werden, die eine berührungslose Temperaturüberwachung der Hautoberfläche des Patienten ermöglichen.

Wie eingangs beschrieben, wird die Kabine durch eine einfache Tür betreten. Die Einrichtung einer Schleuse ist nicht notwendig, da die Kabine während Behandlungspausen auf Leerlaufbetrieb umgeschaltet wird, wobei sich die Kabinenluft erwärmt.

**Patentansprüche**

1. Kabine zur Durchführung der Kryotherapie am ganzen Körper mit einem kalten Behandlungsgas, mit Wänden (1, 2) aus isolierendem Material, einer Tür zum Betreten der Kabine, an den Wänden angeordneten Ausströmvorrichtungen für das Behandlungsgas, in Bodennähe angeordneten Rückführeinrichtungen für das Behandlungsgas, dadurch gekennzeichnet, daß die Kabine oben offen ist und der Abstand von der Oberkante der Wände zum Boden der Kabine auf die Halshöhe des Patienten einstellbar ist.

2. Kabine nach Anspruch 1, dadurch gekennzeichnet, daß der Boden als Gitterrost (4) ausgebildet ist, unter dem eine Absaugöffnung (5) zur Rückführung der Behandlungsgase vorgesehen ist.

3. Kabine nach Anspruch 1 oder 2, dadurch gekennzeichnet, daß die Wände zweiteilig derart ausgeführt sind, daß sie in vertikaler Richtung ineinander geschoben werden können.

4. Kabine nach Anspruch 1 oder 2, dadurch gekennzeichnet, daß der Boden in vertikaler Richtung verstellbar ist.

5. Kabine nach einem der Ansprüche 1 bis 4, dadurch gekennzeichnet, daß die Wände aus doppelwandiger Isolierverglasung aus Kunststoff bestehen.

6. Verfahren zum Betreiben der Kabine nach einem der Ansprüche 1 bis 5, bei dem das Behandlungsgas durch Vermischen von trockener Luft mit einem kalten verflüssigten Gas gebildet wird und das aus der Kabine zurückgeführte Behandlungsgas über einen Wärmeaustauscher (18) geleitet wird, der von warmer trockener Luft zur Bildung des Behandlungsgases durchströmt wird, dadurch gekennzeichnet, daß im Leerlaufbetrieb das Behandlungsgas bereits vor dem Eintritt in das Innere der Kabine umgelenkt und über den Wärmetauscher zurückgeführt wird.

**Claims**

1. Cabin for carrying out crymotherapy over the entire body with a cold treatment gas, having walls (1, 2) made of insulating material, a door for entering the cabin, outflow devices for the treatment gas arranged on the walls, recycling arrangements for the treatment gas arranged in the vicinity of the floor, characterized in that the cabin is open at the top and the distance from the top edge of the walls to the floor of the cabin can be set to the level of the neck of the patient.

2. Cabin according to Claim 1, characterized in that the floor is designed as a grate (4), under which an extraction opening (5) is provided for recycling the treatment gases.

3. Cabin according to Claim 1 or 2, characterized in that the walls are designed in two parts in such a way that they can be pushed into one another in vertical direction.

4. Cabin according to Claim 1 or 2, characterized in that the floor is adjustable in vertical direction.

5. Cabin according to one of Claims 1 to 4, characterized in that the walls consist of double-walled insulating panes made of plastic.

6. Method for operating the cabin according to one of Claims 1 to 5, in which the treatment gas is formed by mixing dry air with a cold liquified gas and the treatment gas recycled from the cabin is fed via a heat exchanger (18), through which warm dry air flows for forming the treatment gas, characterized in that in the idle mode the treatment gas is diverted already before entry into the interior of the cabin and is recycled via the heat exchanger.

## Revendications

1. Cabine pour l'application de la cryothérapie à la totalité du corps avec un gaz de traitement froid, cabine comportant des parois (1, 2) en matériau isolant, une porte pour accéder à la cabine, des dispositifs d'éjection disposés contre les parois pour le gaz de traitement, des dispositifs de recyclage disposés au voisinage du fond pour le gaz de traitement, cabine caractérisée en ce qu'elle est ouverte dans le haut et que la distance entre le bord supérieur des parois et le fond de la cabine est réglable à la hauteur du cou du patient.

2. Cabine selon la revendication 1, caractérisée en ce que le fond revêt la forme d'un caille-botis (4) au-dessous duquel est prévu un orifice d'aspiration (5) pour le recyclage du gaz de traitement.

3. Cabine selon la revendication 1 ou 2, caractérisée en ce que les parois sont réalisées en deux parties de façon qu'elles puissent coulisser l'une dans l'autre en direction verticale.

4. Cabine selon la revendication 1 ou 2, caractérisée en ce que le fond est réglable en direction verticale.

5. Cabine selon une des revendications 1 à 4, caractérisée en ce que les parois sont constituées par un vitrage isolant double en matière plastique.

6. Procédé pour l'exploitation de la cabine selon une des revendications 1 à 5, procédé dans lequel le gaz de traitement est obtenu par mélange d'air sec avec un gaz liquéfié froid, et le gaz de traitement recyclé hors de la cabine passe par un échangeur thermique (18) qui est également parcouru par l'air sec chaud destiné à la formation du gaz de traitement, procédé caractérisé en ce qu'en marche à vide, le gaz de traitement est dévié avant son entrée dans l'intérieur de la cabine et est recyclé par l'intermédiaire de l'échangeur thermique.

Fig.1

Fig.2

Fig.3

Fig. 4